# EUROPEAN PATENT SPECIFICATION

(11) **EP 1 331 938 B1**
(45) Date of publication and mention of the grant of the patent: **25.10.2006**
(21) Application number: 01983031.4
(22) Date of filing: 12.11.2001
(51) Int. Cl.: A61K 35/74, A61P 3/00

(54) **USE OF A STRAIN OF LACTOBACILLUS REDUCING THE RISK FACTORS INVOLVED IN THE METABOLIC SYNDROME**
VERWENDUNG EINES LACTOBACILLUS-STAMMES ZUR VERRINGERUNG DER RISIKOFAKTOREN IM ZUSAMMENHANG MIT DEM METABOLISCHEN SYNDROM
UTILISATION D'UNE SOUCHE DE LACTOBACILLUS REDUISANT LES FACTEURS DE RISQUE ASSOCIES AU SYNDROME METABOLIQUE

(30) Priority: 10.11.2000 SE 0004124
(43) Date of publication of application: 06.08.2003
(73) Proprietor: PROBI AB, 223 70 Lund (SE)
(72) Inventor: JOHANSSON, Marie-Louise, S-222 46 Lund (SE); NARUSZEWICZ, Marek, PL-05-540 Zalesie Gorne (PL)
(74) Representative: Andersson, Björn E.
(86) International application number: PCT/SE2001/002500
(87) International publication number: WO 2002/038165

(56) References cited:
- WO-A1-00/23442
- WO-A1-99/07827
- HANNA BUKOWSKA ET AL.: 'Decrease in fibrinogen and LDL-cholesterol levels upon supplementation of diet with lactobacillus plantarum in subjects with moderately elevated cholesterol' ATHEROSCLEROSIS vol. 137, 1998, pages 437 - 438, XP002909965
- J.C. MOHAN ET AL.: 'Short term hypolipidemic effects of oral lactobacillus sporogenes therapy in patients with primary dyslipidemias' INDIAN HEART JOURNAL vol. 42, no. 5, 1990, pages 361 - 364, XP002909966
- D. ZAPOLSKA-DOWNAR ET AL.: 'Ibuprofen inhibits adhesiveness of monocytes to endothelium and reduces cellular oxidative stress in smokers and non-smokers' EUROPEAN JOURNAL OF CLINICAL INVESTIGATION vol. 30, 2000, pages 1002 - 1010, XP002909967
- M. KAWASE ET AL.: 'Effects of administration of fermented milk containing whey protein concentrate to rats and healthy men on serum lipids and blood pressure' J. DAIRY SCI. vol. 83, 2000, pages 255 - 263, XP002909968
- T. MATSUZAKI ET AL.: 'Antidiabetic effects of an oral administration of lactobacillus casei in a non-insulin-dependent diabetes mellitus (NIDDM) model using KK-AY mice' ENDOCINE JOURNAL vol. 44, no. 3, 1997, pages 357 - 365, XP002909969
- G. MARK BAILLIE ET AL.: 'Insulin and coronary artery disease: is syndrome X the unifying hypothesis?' AMULATORY CARE vol. 32, 1998, pages 233 - 247, XP002909970

## Description

### BACKGROUND OF THE INVENTION

The metabolic syndrome consists of a number of metabolic disorders, many of which promote the development of atherosclerosis and increase the risk of cardiovascular disease. The major components of the metabolic syndrome are atherogenic dyslipidemia, increased blood pressure, elevated plasma glucose, and a protrombotic state. Atherogenic dyslipidemia is manifested as elevated triglycerides, increased small low-density lipoprotein cholesterol (LDL) and decreased high-density lipoprotein cholesterol (HDL). The mechanisms underlying the metabolic syndrome are not fully known, but most patients with the syndrome exhibit a high insulin level and resistance to the cellular actions of insulin.

Patients having abdominal obesity often manifest the multiple risk factors of the metabolic syndrome. Any patient whose triglyceride concentration exceed 150 mg/dl is suspect for the metabolic syndrome. A mild elevation of fasting glucose of 110-125 mg/dl is another clue to the presence of the metabolic syndrome. The first priority in treating the dyslipidemia of the metabolic syndrome should, however, be to lower the atherogenic lipoproteins, such as LDL, which is today most effectively done with statins (Scott M. Grundy, Hypertriglyceridemia, insulin resistance, and the metabolic syndrome, Am J Cardiol 1999;83: 25F-29F).

### PRIOR ART

It has previously been shown that diet supplementation with *Lactobacillus* effectively.reduces fibrinogen levels as well as the level of cholesterol in blood in hypercholesterolemic patients, see WO 99/07827.

The effect of fermented milk containing whey proteins on serum lipids has been investigated and reported in J Dairy Sci 2000 Feb;83(2):255-63. After 8 weeks of consumption the high density lipoprotein cholesterol level showed a significant rise. In addition the atherogenic index, that is (total cholesterol - high density lipoprotein cholesterol)/high-density lipoprotein cholesterol, and the systolic blood pressure were significantly reduced. The tested strains were *Lactobacillus casei* and *Streptococcus thermophilus.*

WO 96/29083 refers to strains of *Lactobacillus plantarum* having a mannose-specific adhesin, especially belonging to a cluster with more than 70% similarity to *L. plantarum* 299, DSM 6595 in terms of REA for the preparation of a pharmaceutical composition inhibiting the binding of pathogenic bacteria expressing mannose-specific adhesins to the epithelial cell surface. *L. plantarum* 299v, DSM 9843, was described as a preferred strain. This cluster seem to comprise all strains of *Lactobacillus plantarum* which have been isolated from human intestinal mucosa.

### DESCRIPTION OF DRAWING

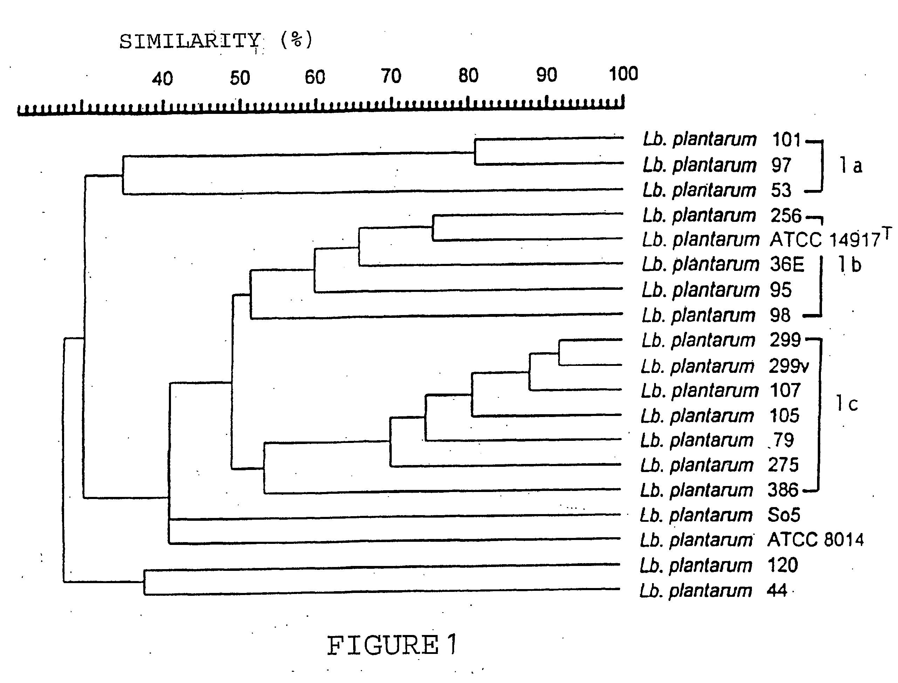

The Figure is a dendrogram showing the similarity in % between different tested strains of Lactobacillus.which have been characterised by the REA-method, based on the Pearson product moment correlation coefficient and UPGMA.

### DESCRIPTION OF THE INVENTION

We have found that in patients with moderately elevated cholesterol levels a supplementation of the diet with ProViva, a functional food product containing fruit juice, fermented oat, and *Lactobacillus plantarum* 299v (DSM 9843), significantly lowers LDL cholesterol and fibrinogen levels, as well as the levels of insulin and leptin, and also the systolic blood pressure, and increases the HDL cholesterol level. This means that most risk factors involved in the metabolic syndrome are affected in a positive way.

It is believed that this effect can be ascribed to the fermentation of the bacteria in the gut giving rise to the short chain fatty acids (SCFA) acetic and propionic acids. Acetic acid and propionic acids are absorbed into the blood, pass into the liver and enter metabolic pathways. It has been postulated that SCFA, mainly.propionic acid, improve glucose tolerance and inhibit cholesterol synthesis in the liver, presumably by inhibiting the rise in free fatty acid levels in serum and by improving insulin sensitivity. It has now been found that said SCFA also have an effect on the expression of PPAR, and an up regulation of PPAR has proven to increase the production of ApoAl, the major constituent of HDL.

In a previous study in healthy adult volunteers administration of a fruit drink containing *Lactobacillus plantarum* 299v (Johansson et al., Int J Food Microbiol 42 (1998) 29-38) was shown to significantly increase the faecal concentration of short chain fatty acids, and especially of propionic acid and acetic acid. This effect could be explained as 299v stimulating the bacterial flora in colon to produce acetic and propionic acid. However, not all strains of *Lactobacillus* triggers this increase in short chain fatty acids. In a later study (Molin et al., in manuscript) it has been shown that *Lactobacillus rhamnosus* 271 (DSM 6594) as well as a *Streptococcus theromophilus* strain do not give rise to this increase of SCFA in faeces. Nor does a tested strain of *Lactobacillus acidophilus.*

That propionic acid has an effect on the cholesterol level has also been demonstrated (Zapolska-Downar et al., Eur J Clin Invest 2000; 30:1-3) in a clinical trial with ibuprofen, a propionic acid derivative. In addition to the reduction of cellular oxidative stress factors and inhibition of adhesiveness of monocytes to endothelium it was also found that the HDL cholesterol levels were increased.

The present invention thus refers to the use of a strain of *Lactobacillus* giving rise to increased amounts of propionic acid or acetic acid in colon for the preparation of a medicament reducing one or more of the risk factors involved in the metabolic syndrome, including increased blood pressure, decreased HDL, increased LDL, high insulin level, and high leptin level in blood.

The invention especially refers to the use of a strain of *Lactobacillus* having the ability to reduce the blood pressure, to increase the HDL, and to reduce the LDL, the insulin level, and the leptin level in blood.

A preferred strain of *Lactobacillus* to be used according to the invention is *Lactobacillus plantarum.*

Another preferred property of the strains to be used according to the invention is the ability to bind to the intestinal mucosa. Two factors seem to be crucial for the exertion of ecological effects of lactobacilli. The first is the capacity to colonise the intestine, that is to survive in high numbers for a period of time after the last administration of live bacteria. This property may be important for the ability of the lactobacilli to suppress the growth and proliferation of pathogenic bacteria, but not sufficient. The second is the capacity to bind directly to intestinal epithelial cells or mucins. This may be one of the factors that promotes colonisation, but is not a prerequisite for colonisation.

Strains of *Lactobacillus* have been shown to increase the production of intestinal mucins, and it is believed that said mucins neutralise pathogens and improve the propagation of bacteria. In a preferred aspect of the invention the strain of *Lactobacillus* to be used according to the invention should also have the ability to increase the production of mucin in colon.

The invention also refers to a strain of *Lactobacillus plantarum* belonging to the cluster having a restriction endonuclease analysis similarity of more than 55% to the strain *Lactobacillus plantarum* 299, deposition number DSM 6595, by using the Pearson product moment correlation coefficient and the unweighted pair group algorithm with arithmetic averages (UPGMA; GelCompare 3.0, Applied Maths, Kortrijk, Belgium) for the preparation of a medicament reducing one or more of the risk factors involved in the metabolic syndrome, including increased blood pressure, decreased HDL, increased LDL, high insulin level, and high leptin level in blood. Said strains in a preferred aspect of the invention have the ability to reduce the blood pressure, to increase the HDL, and to reduce the LDL, the insulin level; and the leptin level in blood. The borderline between a similarity of 55 % and 70 % is indistinct. A majority of the strains from human instestinal mucosa have a similarity within 70 %, but for instance *Lactobacillus plantarum* 386, with a similarity within 55 %, has also been isolated from human intestines and presents the same binding mechanisms as the other strains of the cluster.

Examples of said preferred strains of *Lactobacillus plantarum* are shown in the Figure, that is: *Lactobacillus plantarum* 299, *Lactobacillus plantarum* 299v, *Lactobacillus plantarum* 107, *Lactobacillus plantarum* 105, *Lactobacillus plantarum* 79, *Lactobacillus plantarum* 275, and *Lactobacillus plantarum* 386. ATCC 14917^{T} denotes the type strain for *Lactobacillus plantarum.*

The strains *Lactobacillus plantarum* 299 and 299v, which were both isolated from healthy human intestinal mucosa, have been deposited at the Deutsche Sammlung von Mikroorganismen und Zellkulturen GmbH on July 2, 1991 and March 16, 1995, respectively, and have been given the deposition numbers DSM 6595 (299) and DSM 9843 (299v).

The invention in a preferred aspect refers to the use of the strain *Lactobacillus plantarum* 299v, DSM 9843.

The bacteria to be used according to the invention can be administered in a conventional carrier for a medicament or food product to be delivered to the intestines, such as a physiologically acceptable substrate which can be fermented by the bacterium in question, as well as foodstuffs of various kinds, especially based on starch or milk, but also inert solid or liquid substances, such as saline or water. A suitable substrate should contain liquid or solid fibres which are not resorbed in the gastro-intestinal tract and which when fermented with *Lactobacillus* form carboxylic acids. As an example of suitable, starch-containing substrates can be mentioned cereals, such as oats and wheat, corn, root vegetables such as potatoes and certain fruits such as green bananas. A preferred substrate for the use according to the invention, which also gives the composition an excellent nutritional value, is a nutrient solution based on oatmeal, for instance as described in WO 89/08405. Tests have shown that the effect of the *Lactobacillus* strains is improved if dietary fibres, for instance in the form of oatmeal gruel or of - glucans, are supplied.

The invention especially refers to the use of the strain of *Lactobacillus* in combination with dietary fibres.

The bacteria to be used according to the invention can be administered in any suitable way, preferably orally or rectally, for example in the form of enema. They can also be administered enterally through a catheter inserted in the intestines via the stomach or directly in the intestines.

The bacteria should be provided in a therapeutically effective dose, said dose could be from 10⁸, preferably not less than 1x10¹⁰ CFU/d

According to the invention the described strains of *Lactobacillus* can be used for the preparation of a medicament increasing the level of HDL cholesterol in serum; or for the preparation of a medicament reducing a high systolic blood pressure; or for the preparation of a medicament decreasing the insulin level in serum; or for the preparation of a medicament reducing the leptin level in serum.

### EXAMPLES

### Example 1. Effect of propionic acid on PPAR gene expression in vitro

This experiment was done to investigate the effect of short chain fatty acids on the production of PPAR.

Human umbilical vein endothelial cells, HUVEC, were obtained from umbilical cords by collagenase digestion as described by Jaffe et al.(Biology of Endothelial Cells, Boston, Martinus Nijhoff Publishers, 1984, p. 1-13). In brief, veins of umbilical cords were perfused with PBS to remove blood cells, filled with 0.1 % collagenase (type Ia) and left for 10 minutes at 37°C. Suspended HUVEC were supplemented with PBS, centrifuged and cultured at 37°C in gelatine-coated 25 ml flasks and 6-well tissue culture plates filled with medium 199, under humidified 5% CO₂ in room air. Propionic acid was added to the culture medium in the amounts of 0.1, 1.0, and 10.0 mmol/l, and the effect of the acid on the expression of PPAR was determined.

Total RNA was extracted from the cells by the method of Chomczynski using TRIZOL (Gibco BRL). After isolation the integrity of the RNA samples was checked by gel electrophoresis in 1% agarose gel stained with ethidium bromide. The concentration of total RNA was calculated after spectrophotometric measurements at 260 nm wavelength. Each RNA sample (500 ng) was incubated with 1 U of DNAse I (Boehringer Mannheim, Germany) for 15 minutes at 37°C, and subsequently DNAse I was inactivated by heating at 75°C for 3 minutes. Such DNAse-treated RNA (500 ng) was dissolved in 20 µl of a reaction mixture containing 2.5 mM of dATP, dTTP, dCTP, and dGTP (Progmega, USA), 20 U of RNAsin (Promega, USA), 100 pM of randon hexamers (Boehringer Mannheim, Germany) and 20 U of MMLV Reverse Transcriptase (Boehringer Mannheim). Incubation was carried out at 37°C for 60 minutes; the temperature of the reaction was then raised to 94°C for 5 minutes to inactivate the enzyme and finally dropped to 4°C. An aliquot of cDNA (5 µl of RT mixture, cDNA resulting from transcription of 125 ng RNA) was dissolved in 40 µl of a reaction mixture containing 10 x PCR buffer (final Mg²⁺ concentration 1.5 mM, Boehringer Mannheim), 2.5 mM of dATP, dTTP, dCTP, and dGTP, 10 pM of up- and downstream primers (PPAR and GAPDH set, respectively) and 1 U Taq polymerase (Boehringer Mannheim). The amplification profile consisted of an initial denaturation at 94°C for 3 minutes, followed by denaturation at 94°C for 30 seconds, annealing at 57°C for 1 minute (both for PPAR and GAPDH) and extension at 72°C for 1 minute. For semiquantitative analysis, linearity of amplification of PPAR and GAPDH cDNAs depending on PCR cycle number was established in preliminary experiments.

The following sets of primers were used in the PCR amplification:
PPAR
   sense 5'-GCCCCTCCTCGGTGACTTATC-3'
   antisense 5'-ATGACCCGGGCTTTGACCTT-3'
GAPDH
   sense 5'-GAGTCAACGGATTTGGTCGT-3'
   antisense 5'-GTTGTCATGGATGACCTTGG-3'
Amplification products obtained by PCR (PPAR cDNA of 454 bp in length and GAPDH cDNA of 482 bp in length) were electrophoretically separated on 3% agarose gel. Images of ethidium bromide-stained bands for PPAR and GAPDH cDNAs were photographed with DS-34 Polaroid camera. The intensity of the bands was densitometrically measured with the gel analysis macro supplied with NIH Image. All PPAR signals were normalised to mRNA levels of the housekeeping gene GAPDH and expressed as a ratio.

The results are given in Table 1 below.

**Table 1. Effect of propionic acid on the expression of PPAR mRNA in human endothelial cells**

| | Concentration of propionic acid, mmol/l | | |
|---|---|---|---|
| Increase of mRNA, % | 0.1 | 1.0 | 10.0 |
| | 29.3 | 47.2 | 89.7 |

Data are expressed as percent in relation to GAPDH mRNA (100%).

If propionic acid is exchanged for acetic acid in the above Example 1, similar results are obtained.

### Example 2. Study of metabolic risk factors in healthy smokers

A study was performed in order to investigate the effect of ProViva (Skånemejerier, Malmö. Sweden), a rosehip drink (consisting of 95:5 v/v fruit drink: fermented oatmeal soup) containing *Lactobacillus plantarum* 299v in an amount of 5x10⁷ cfu/ml and oat fibre in an amount of 0.08 g/100 ml, on the levels of fibrinogen, cholesterol, leptin and insulin in blood serum. 19 men and 19 women, healthy smokers aged 35-45 with moderately elevated fibrinogen and cholesterol levels, were randomly divided into two groups A (n=18) and B (n=20). Group A was given 400 ml ProViva per day, 200 ml in the morning and 200 ml in the evening, and group B was given the same amount of placebo, that is the rosehip drink without fermented oats. The experiment lasted for 6 weeks with no change in lifestyle.

Blood was drawn and blood pressure measured before the start of the experiment and after 6 weeks.

After six weeks of administration a significant reduction (p<0.001) in systolic blood pressure was observed, see Table 2. This decrease was most evident in patients with higher baseline levels of systolic blood pressure, and could not be detected in the placebo group. The data given in the table are mean values ± SD.

BMI remained at roughly the same level in both groups during the experiment.

**Table 2. Clinical characteristics before and after administration of ProViva**

| PARAMETER | ProViva | | Placebo | |
|---|---|---|---|---|
| | before | after 6 weeks | before | after 6 weeks |
| Subjects, n (women/men) | 18 (7/11) | 18 (7/11) | 20 (12/8) | 20 (12/8) |
| Age, years | 42.3 ± 2.8 | | 40.1 ± 3.7 | |
| BMI, kg/m2 | 24.8 ± 4.8 | 25.2 ± 4.8 | 26.2 ± 4.2 | 26.3 ± 4.1 |
| SBP, mm Hg | 134 ± 20 | 121 ± 16* | 127 ± 15 | 123 ± 18 |
| | | | | |
| DBP, mm Hg | 89 ± 13 | 84 ± 16 | 87 ± 10 | 83 ± 11 |

| | | | | |
|---|---|---|---|---|
| * p<0.001 | | | | |

Cholesterol and triglyceride levels in serum were determined using enzyme kits (CHOD-PAP, GPO-PAP). HDL cholesterol was measured after precipitation of lipoproteins containing apoB with phosphotungstic acid in the presence of Mg²⁺. LDL-cholesterol was determined after precipitation of LDL with polyvinyl sulphate. Laboratory procedures were based on test kits from Boehringer Mannheim. Glucose was measured using glucose oxidase and test kits from Analco (PAP) and plasma fibrinogen determinations followed the method of Clauss based on thrombin time (test kits from bioMérieux). Insulin was measured by the Abbott Imx Insulin assay (Abbott Laboratories, Tokyo, Japan) and leptin was quantified in serum by means of the Human Leptin Ria kit (from Linco Research Inc., Charles, MD, USA).

No change in plasma levels of total cholesterol, triglycerides could be observed. Patients in group A, however, showed a 12 % decrease in LDL cholesterol and a 10 % increase in HDL cholesterol, see Table 3.

The fibrinogen level was reduced by about 10 %.

A significant reduction in insulin levels and a 37 % reduction in leptin concentration were also found.

**Table 3. Effect of administration of ProViva on biochemical parameters**

| PARAMETER | ProViva | | Placebo | |
|---|---|---|---|---|
| | before | after 6 weeks | before | after 6 weeks |
| Triglycerides, mg/dl | 121 ± 38 | 129 ± 48 | 141 ± 70 | 131 ±67 |
| Cholesterol, mg/dl | 216 ± 34 | 213 ± 33 | 212 ± 30 | 212 ± 41 |
| LDL-cholesterol, mg/dl | 138 ± 37 | 122 ± 36* | 13.5 ± 35 | 129 ± 46 |
| HDL-cholesterol, mg/dl | 45 ± 8 | 50 ± 9* | 47 ± 14 | 49 ± 15 |
| Glucose, mg/dl | 110 ± 22 | 110 ± 14 | 106 ± 13 | 109 ± 13 |
| Insulin, U/ml | 11.1±5.7 | 7.9±4.3* | 7.7±4.7 | 7.6±3.2 |
| Leptin, ng/ml | 13.0±7.7 | 8.2±4.6** | 17.4±6.4. | 18.3±7.2 |
| Fibrinogen, mg/dl | 380 ± 37 | 301 ± 33 | 362 ± 38 | 390 ± 46 |

| | | | | |
|---|---|---|---|---|
| * p<0.05 ** p<0.001 | | | | |

### Example 3. Study of metabolic risk factors in psoriasis patients

In this study the effect of the administration of a concentrated oatmeal gruel fermented with *Lactobacillus plantarum* 299v, DSM 9843, to a group of 6 patients with relapsing psoriasis on different factors was investigated. The patients were untreated, that is without medication for as least 6 weeks before the start of the administration of the.concentrated oatmeal gruel, containing 1x10⁹ cfu/ml *L. plantarum*. Each patient was given 50 ml/d for 12 weeks.

The levels of HDL and LDL before the administration and after 6 weeks were determined. The results are given in the following Table 4.

**Table 4. Levels of LDL and HDL cholesterol after administration of L. plantarum 299v**

| | *L*. *pl.* 299v (1x10⁹ cfu/ml), 50 ml/d | | Placebo | |
|---|---|---|---|---|
| | before | after 12 weeks | before | after 12 weeks |
| LDL cholesterol, mg/dl | 149.2±11 | 135.4±8.5* | 146.1±7.2 | 139.5±6.7 |
| HDL cholesterol, mg/dl | 41.6±8.3 | 52.5±9.0** | 43.8±7.5 | 41.5±6.7 |

| | | | | |
|---|---|---|---|---|
| * p < 0.05 ** p < 0.001 | | | | |

Psoriasis patients in general have low HDL values and therefore are at a greater risk of suffering from the metabolic syndrome. This study shows that this risk can be reduced by the intake of the bacterium *Lactobacillus plantarum* 299v.

## Claims

1. Use of a strain of Lactobacillus plantarum for the preparation of a medicament for reducing the risk factors involved in the metabolic syndrome, including increased blood pressure, high insulin level, and high leptin level.

2. Use according to claim 1 of a strain of Lactobacillus plantarum having the ability to bind to the intestinal mucosa.

3. Use according to claim 1 or 2, wherein the strain is Lactobacillus plantarum 299v, DSM 9843.

4. Use according to any of claims 1-3, wherein the strain of Lactobacillus plantarum is used in combination with dietary fibres.

## Patentansprüche

1. Verwendung eines Stammes von Lactobacillus plantarum zur Herstellung eines Medikaments zur Verringerung der Risikofaktoren im Zusammenhang mit dem metabolischen Syndrom, einschließlich erhöhten Blutdrucks, hohen Insulinspiegels und hohen Leptinspiegels.

2. Verwendung eines Stammes von Lactobacillus plantarum, der die Fähigkeit, an die Darmmucosa zu binden, besitzt, nach Anspruch 1.

3. Verwendung nach Anspruch 1 oder 2, wobei es sich bei dem Stamm um Lactobacillus plantarum 299v, DSM 9843, handelt.

4. Verwendung nach einem der Ansprüche 1-3, wobei der Stamm von Lactobacillus plantarum in Verbindung mit Nahrungsfasern verwendet wird.

## Revendications

1. Utilisation d'une souche de Lactobacillus plantarum pour la préparation d'un médicament destiné à réduire les facteurs de risque impliqués dans le syndrome métabolique, comprenant une tension artérielle accrue, un taux d'insuline élevé et un taux de leptine élevé.

2. Utilisation selon la revendication 1 d'une souche de Lactobacillus plantarum ayant la capacité de se lier à la muqueuse intestinale.

3. Utilisation selon la revendication 1 ou 2, dans laquelle la souche est Lactobacillus plantarum 299v, DSM 9843.

4. Utilisation selon l'une quelconque des revendications 1 à 3, dans laquelle la souche de Lactobacillus plantarum est utilisée en combinaison avec des fibres alimentaires.
